# EUROPEAN PATENT APPLICATION

(11) **EP 1 158 045 A1**
(43) Date of publication of application: **28.11.2001**
(21) Application number: 00870108.8
(22) Date of filing: 17.05.2000
(51) Int. Cl.: C12N 5/08, G01N 33/50

(54) **Culture conditions allowing to modulate the expression of CYP3A4 in CaCo2 cells**

(71) Applicant: UNIVERSITE CATHOLIQUE DE LOUVAIN, 1348 Louvain-la-Neuve (BE)
(72) Inventor: Schneider, Yves-Jacques, 5500 Flamignoul (BE); Burteau, Nathalie, 6001 Marcinelle (BE)
(74) Representative: De Clercq, Ann

(57) **Abstract**

The present invention relates to the field of the expression of biotransformation activities in cell culture systems. The present invention discloses basal and well defined culture conditions for CaCo 2 cells and therefore provide for *in vitro* model systems of the intestinal barrier as a tool in pharmaco-toxicology. In these model systems are most, if not all, biotransformation activities expressed *in vitro,* present, providing a model mimicking the *in vivo* reality. Under the culture conditions disclosed by the present invention, the expression of these activities, and especially the expression of CYP3A4, does not rely upon the addition of any inducer for CYP3A4 expression. These culture conditions further comprise the use of permeable culture inserts containing either a polyethylene terephtalate (PET) or an Anopore™ membrane.

Furthermore, the present invention discloses CaCo 2 cell lines which show an enhanced expression of CYP3A4 in comparison with their parental cell lines, when grown without the addition of an inducer for CYP3A4 expression.

## Description

### Field of the invention

The present invention relates to the field of the expression of biotransformation activities in cell culture systems as *in vitro* model of the intestinal barrier as a tool in pharmaco-toxicology and, more precisely, CYP3A4 in human CaCo 2 cells.

### Background of the invention

CYP3A4 is the major isoform of cytochrome P450 expressed in human liver (Shimada and Guengerich, 1989) and enterocytes (Watkins *et al.,* 1987; Kolars *et al.,* 1992). It plays an important role in the metabolisation of *ca.* 50 % of drugs given orally to patients as well as of numerous xenobiotics or substances present in the environment.

Cell culture systems based on the use of human intestinal CaCo 2 cells are increasingly used in order to evaluate the intestinal permeability of various substances (Artursson *et al.,* 1996; Bailey *et al.,* 1996). Although CaCo 2 cells are derived from a colon adenocarcinoma, there is a good correlation between the passage of many substances across monolayers of differentiated CaCo 2 cells and the intestine, *in vivo* or as isolated organ (Hillgren *et al.,* 1995). Furthermore, CaCo 2 cells express many characteristics of enterocytes and, in particular, transport systems, brush border enzymes, tight junctions, ... as well as extrusion systems such as P-glycoprotein and multidrug resistence associated proteins (MRPs).

Nevertheless, among a few others, the main limitation which restricts the predictive value of this *in vitro* model of the intestinal barrier is the absence of expression of CYP3A4 (Gan *et al.,* 1996). This impairs to take into account the biotransformation of substances by this isoform of cytochrome P450 as well as possible inhibition/induction of the enzyme, which seems critical in the case of possible drug interactions (Lown *et al.,* 1997).

In order to solve the problem of the absence of CYP3A4, different approaches have been developed.
- CaCo 2 cells have been transfected with a vector that cause the expression of CYP3A4 in these cells (Crespi *et al.,* 1996; Hu *et al.,* 1999; Patent WO9708342, 1997) but this mutation appears unstable and the vector is not passed efficiently from one cell generation to the next one. CYP3A4 activities can be "stabilized" but with addition of 2'-deoxy-5-azacytidine in the culture medium of the cells.
- TC7 cells, a clone of the CaCo 2 line, has been isolated. These cells contain a large amount of CYP3A but which appears to be the CYP3A5 isoform (Carrière *et al*., 1994) which is not the main isoform expressed in enterocytes and, furthermore, which is not inducible.
- CaCo 2 cells have been cultivated in a culture medium containing serum in the presence of derivatives of vitamin D3, such as 25-hydoxyvitamin D3 or 1α, 25-dihydroxyvitamin D3 which induces the expression of CYP3A4 in CaCo 2 cells (Schmiedlin-Ren *et al.,* 1997; Patent US 58566189, 1999) But these derivatives of vitamin D3 seem implicated in apoptosis process (Skarosi *et al.,* 1997). More, the addition of this inducer is expensive, which is a disadvantage if CaCo 2 cells or clones are used to screen a lot of compounds.

The aim of the present invention is to provide cells, cell lines and clones showing enhanced and stable expression of CYP3A4.

It is further an aim of the invention to provide methods for growing these cells, cell lines and clones.

It is another aim of the invention to provide a method for growing CaCo2 cells with enhanced and stable but still inducible expression of CYP3A4.

It is also an aim of the invention to provide systems for screening the bioavailability of pharmaceutical compounds, xenobiotics and environmental pollutants.

### Summary of the invention

The present invention discloses basal and well defined culture conditions for CaCo 2 cells and therefore provide for a cell culture system wherein most, if not all, biotransformation activities expressed in the small intestine are present *in vitro,* providing a model mimicking the *in vivo* reality. Under the culture conditions disclosed by the present invention, the expression of these activities, and especially the expression of CYP3A4, does not rely upon the addition of any inducer for CYP3A4 expression. These culture conditions further comprise under preferred embodiments the use of permeable culture inserts containing either a polyethylene terephtalate (PET) or an Anopore™ membrane.

Furthermore, the present invention discloses CaCo 2 cell lines which show an enhanced expression of CYP3A4 in comparison with their parental cell lines, when grown without the addition of an inducer for CYP3A4 expression. However, the expression of CYP3A4 by these cells can still be enhanced by the addition of certain vitamin D3 analogues, specifically 25-hydroxyvitamin D3 and 1-α-25-dihydroxyvitamin D3, or by the addition of sodium butyrate.

### Detailed description of the invention

According to a first embodiment, the present invention relates to CaCo 2 cell lines showing an enhanced expression of CYP3A4 without the addition of an inducer for CYP3A4 expression.

The expression "CaCo 2 cell line" refers to a cell line derived from a human colon carcinoma cell line. In the invention, The CaCo 2 cells "late passages" (noted as CaCo 2 L and used from passage 182 to 208) or "early passages" (noted as CaCo 2 E and used from passage 68 to 91) of the invention were derived from the CaCo 2 cell line with ATCC Accession number HTB-37. Using the limiting dilution technique, the inventors surprisingly were able to identify clones which express more CYP3A4 in the absence of induction than the parental cell lines (L and E).

As thus, the expression "showing an enhanced expression of CYP3A4 without the addition of an inducer for CYP3A4 expression" can be evaluated by comparing the expression of CYP3A4 by candidate CaCo 2 cells and cell lines with the level of CYP3A4 expression of the parental CaCo2 cell line (ATCC HTB-37) as indicated above. The expression of CYP3A4 can be monitored for instance by following CYP3A4 mRNA expression or by using antibodies against CYP3A as is further specified in the examples section, but also by all other techniques as known by the person skilled in the art to evaluate expression of genes and proteins.

One of these CaCo 2 cell lines showing enhanced expression of CYP3A4, further specified as CaCo 2-BT-01 has been deposited on April, 10, 2000 at the Belgian Coordinated Collection of Microorganisms (LMBP).

The expression "inducer for CYP3A4 expression" relates to a derivative of vitamin D3 or to sodium butyrate or any other known inducer for CYP3A4 known in the art. Derivatives of vitamin D3 having the property of inducing CYP3A4 expression are for instance compounds derived from cholecalciferol (vitamin D3) including, but not limited to 25-hydroxyvitamin D3 (calcifediol) and 1-α-25-dihydroxyvitamin D3 (calcitriol), as well as compounds which may be produced, for example, by the addition of alkyl, alkoxy, hydroxyl groups, etc. to vitamin D3 to produce compounds that function in a manner similar (e.g. contribute to enhanced catalytic activity) to 25-hydroxyvitamin D3 and 1-α-25-dihydroxyvitamin D3.

Accordingly, the present invention also relates to a CaCo 2 cell line as identified above showing an enhanced expression of CYP3A4 characterized in that it reaches at least the same level of CYP3A4 expression of the cell line deposited on April, 10, 2000 at the Belgian Coordinated Collection of Microorganisms (LMBP).

The CaCo 2 cell lines according to this embodiment can be evaluated by comparing the expression level of CYP3A4 by candidate CaCo 2 cells and cell lines with the level of CYP3A4 expression of the cell line deposited on April, 10, 2000 at the Belgian Coordinated Collection of Microorganisms (LMBP) when grown without the addition of an inducer for CYP3A4 expression. The expression of CYP3A4 can be monitored for instance by following CYP3A4 mRNA expression or by using antibodies against CYP3A, but also by all other techniques known by the person skilled in the art to evaluate expression of genes and proteins.

In a preferred embodiment, the invention relates to a CaCo 2 cell line, named CaCo 2-BT-01 and deposited on April, 10, 2000 at the Belgian Coordinated Collection of Microorganisms (LMBP) and showing an enhanced expression of CYP3A4 without the addition of an inducer for CYP3A4 expression.

According to another embodiment, the present invention relates to a method of growing the above-mentioned CaCo2 cells and cell lines comprising growing said cells without the addition of an inducer for CYP3A4 expression.

According to another embodiment, the invention relates to a method of growing CaCo2 cells comprising the steps of:
- providing at least:
   a) a CaCo 2 cell line according to any of claims 1 to 3, and,
   b) a growth medium for cultivation of said CaCo 2 cells comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and,
- seeding said cells onto a permeable culture insert.

In a preferred embodiment, this permeable culture insert is chosen from PET microporous membranes having a pore size of 0,4 to 3 µm and Anopore™ membranes having a pore size of 0,02 to 0,2 µm

According to another embodiment, the present invention relates to a method of growing CaCo 2 cells to allow for the enhanced expression of CYP3A4 without any addition of an inducer for CYP3A4 expression comprising growing said cells on a permeable culture insert chosen from PET microporous membranes having a pore size of 0.4 to 3 µm and an Anopore™ membranes having a pore size of 0.02 to 0.2 µm.

The present invention further relates to a method of growing CaCo 2 cells to allow for the enhanced expression of CYP3A4 according to claim 7 comprising the steps of:
- providing a growth medium for cultivation of said CaCo 2 cells comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and
- seeding said cells on a permeable culture insert.

In a preferred embodiment, the permeable culture insert is chosen from PET microporous membranes having a pore size of 0.4 to 3 µm and an Anopore™ membranes having a pore size of 0.02 to 0.2 µm

The growth medium for cultivation of the CaCo 2 cells is described in the examples as the synthetic BDM (basal defined medium) containing all essentially required components. It should be clear that the exact concentrations and amounts of the compounds as specified above are still susceptible for minor changes with regard to the upper limits.

As presented in the current invention, it is a major advantage to be able to grow and differentiate CaCo 2 cells according to the above specified method in a nutritive medium which is totally synthetic and in the absence of poorly defined constituents such as serum, proteins and substances from animal origin. Such a medium has a cost advantage effect but, more important, is devoid of additives such as, for instance serum with variable composition and multiple effects on inducible genes as well as contamination problems. The present inventors surprisingly found that under these basal and well defined culture conditions it is possible to obtain expression of CYP3A4 without the addition of any inducer such as vitamin D3 derivative, which is known to affect also many other genes.

The term "permeable culture insert" refers to a cell support device which generally comprises a membrane that supports cell growth. In particular embodiments, the culture inserts used in the present invention comprise a "permeable" microporous membrane that allows free diffusion of ions and macromolecules; as a result, cells grown on such inserts generally mimic the *in* *vivo* situation more closely than cells grown on solid surfaces. Routinely, all the membranes are coated with an extracellular matrix, usually type I collagen, but other commercial coatings of membranes, such as unpolymerised or fibrilar collagen type I, collagen type IV, laminin, matrigel, growth factor reduced matrigel an other extra cellular matrices known in the art can be used as well. In the present invention it has been demonstrated that the CaCo 2 cells may also be cultivated in the absence of collagen coating (data not shown).

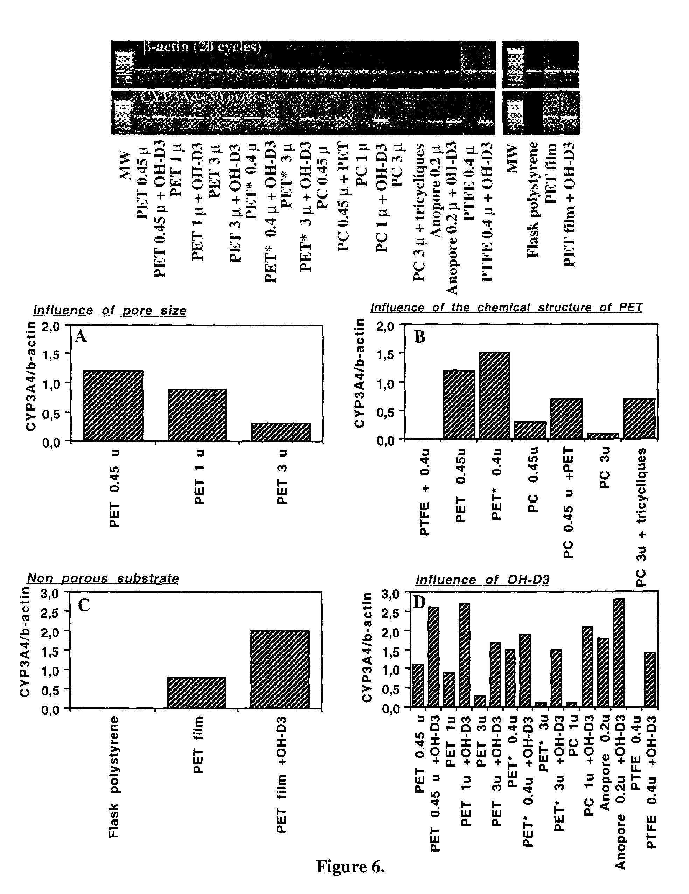

Additionally, the present inventors demonstrated (see Example 4 and figure 6) that the expression of CYP3A4 in CaCo 2 cells is clearly related to the chemical nature of the membranes of the culture insert. No CYP3A4 mRNA is detected when these cells are cultivated up to confluence in tissue culture polystyrene flasks or when they are cultivated during 3 weeks in inserts with polytetrafluoroethylene (PTFE) membrane. A low level of expression is found in cells differentiated for 3 weeks on polycarbonate (PC) track etched membranes. A surprisingly high level of expression is detected in cells cultivated in the same conditions on polyethylene terephtalate (PET) track etched membranes or on Anopore™ inorganic membranes. Furthermore, the diameter of the pores of the membrane seems also to affect of CYP3A4 expression. Therefore it is preferred to use pore sizes of the PET membranes of 0.4, 0.45, 0.9 or 1 µm and a pore size of 0.2 µm for the Anopore™ membrane in any of the methods of the present invention.

Furthermore, the culture conditions provided in these methods, allow the expression of most of the activities involved in the intestinal biotransformations, regulating the bioavailability of drugs and xenobiotics, among which the CYP3A4 enzyme plays a key role.

Therefore the present invention also relates to an *in vitro* model for screening the bioavailability of pharmaceutical compounds, xenobiotics or environmental pollutants, comprising a CaCo 2 cell line according to any of claims 1 to 3, seeded onto a permeable culture insert.

The term "xenobiotic" relates to a drug or other foreign substance capable of harming or affecting a living organism or cell.

The expression "screening the bioavailability of" refers to the process of introducing compounds into a cell culture model that has effects representative of the first-pass effect observed in human subjects. In other words, the cell culture model provides information regarding the extent to which the compound will reach its site of action following oral administration in humans. It is contemplated that a large number of compounds can be tested using the cell culture model of the present invention. Those compounds with favourable bioavailability characteristics (e.g. with respect to 'pharmaceutical compounds' :an effective amount of the compound reproducibly enters the systemic circulation) may then be evaluated further.

The present invention also relates to an *in vitro* model for screening the bioavailability of pharmaceutical compounds, xenobiotics or environmental pollutants comprising:
a) a CaCo 2 cell line according to any of claims 1 to 3,
b) possibly a mucus secreting cell line, and
c) a growth medium comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and,
d) a permeable culture insert.

According to a preferred embodiment, the culture insert used in the *in vitro* model of claims 9 and 10 can be chosen from PET microporous membranes having a pore size of 0.4 to 3 µm and Anopore™ membrane having a pore size of 0.02 to 0.2 µm.

The invention also relates to systems wherein co-cultures of intestinal cells are grown in order to obtain an improved *in vitro* model of the intestinal barrier. Indeed, *in vivo*, the small intestine is formed by different cell types, in particular enterocytes or absorptive cells (mimicked *in vitro* by CaCo 2 cells) and mucus secreting goblet cells (mimicked *in vitro* by HT29 cells). The present invention thus also relates to *in vitro* models consisting of mixtures of cells of these two cell types.

The expression "a mucus secreting cell line" refers to, for instance, some clones of HT 29 cells (ATCC HTB-38), LS 174T cells (ATCC CL-188), T84 cells and HCT8 cells.

According to another embodiment, the present invention relates to an *in vitro* model for screening the bioavailability of pharmaceutical compounds, xenobiotics or environmental pollutants comprising:
a) a CaCo 2 cell line,
b) possibly providing a mucus secreting cell line, and,
c) providing a method of growing said CaCo 2 cells to allow for the enhanced expression of CYP3A4 according to claim 7 or 8.

In the *in vitro* models as described above, the expression of CYP3A4 can be further enhanced by the addition of an inducer for CYP3A4 expression. Therefore, the invention also relates to an *in vitro* model according to any of claims 9 to 12 further comprising the use of an inducer for CYP3A4 expression.

According to yet another embodiment, the invention relates to a method of growing CaCo 2 cells of any of claims 1 to 3 comprising treating said cells with an inducer for CYP3A4 expression.

The invention further relates to a method of growing CaCo 2 cells comprising the steps of:
- providing at least:
   a) a CaCo 2 cell line according to any of claims 1 to 3,
   b) possibly a mucus secreting cell line, and,
   c) an inducer of CYP3A4 expression, and,
- seeding said cells onto a permeable culture insert, and,
- treating said cells with said inducer.

According to a preferred embodiment, the permeable culture insert of the above-mentioned method is chosen from PET microporous membranes having a pore size of 0.4 to 3 µm and Anopore™ membranes having a pore size of 0.02 to 0.2 µm.

According to the invention, the inducer in any of the methods of the invention is chosen from a vitamin D3 derivative or sodium butyrate.

The invention, now being generally described, will be more readily understood by reference to the following tables, figures and examples, which are included merely for purposes of illustration of certain aspects and embodiments of the present invention and are not intended to limit the invention. All of the references_mentioned herein are incorporated by reference.

### BRIEF DESCRIPTION OF TABLES AND FIGURES

***Table 1:*** Sequence of primers used for RT-PCR. For each mRNA tested, the table gives the molecular weight (MW) of the PCR product, the sequence of the primer for the 3' and for the 5' end of the DNA chain, the number of cycles (N° cycles) and the hybridisation temperature (Tₕ) used in the PCR experiments.

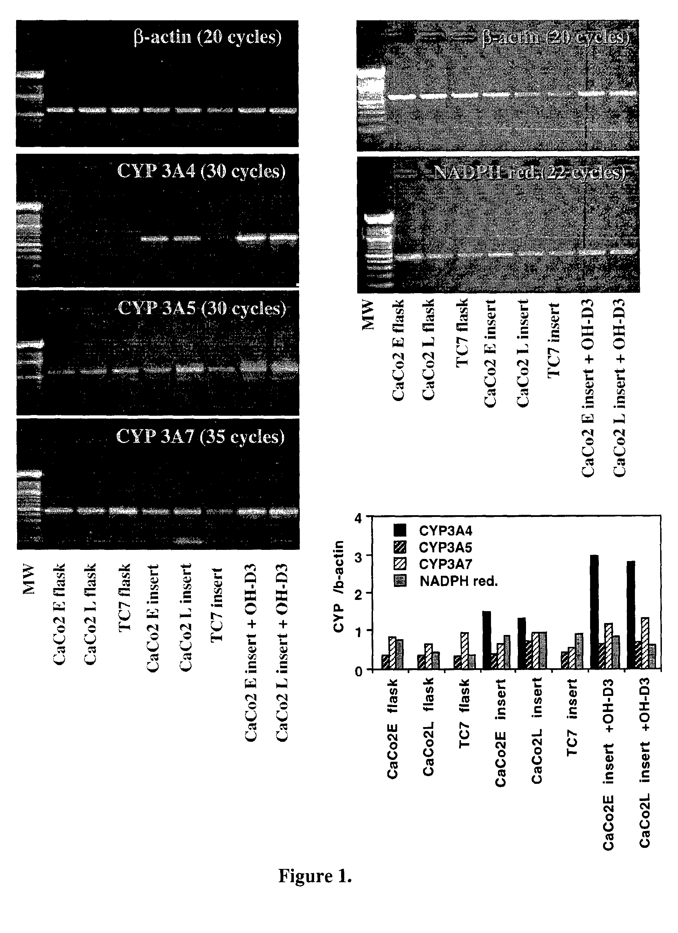

***Figure 1:*** Expression of mRNA for CYP3A4, CYP3A5, CYP3A7, NADPH cytochrome P450 reductase and β-actin in CaCo 2 cells and TC7 cells cultivated in different conditions.
   Specific primers for CYP3A4, CYP3A5, CYP3A7 and NADPH cytochrome P450 reductase were used for a RT-PCR experiment on cDNA from CaCo 2 E, CaCo 2 L and TC7 cells grown on flasks or on PET inserts in synthetic BDM medium in the presence or absence of 2.5 µM 25-hydroxyvitamin D3. Ethidium bromide stained agarose gels were analysed with NIH Image with "gel plotting" macro: intensity of each band was divided by the intensity of the corresponding band of β-actin and results were collected in the graph enclosed.

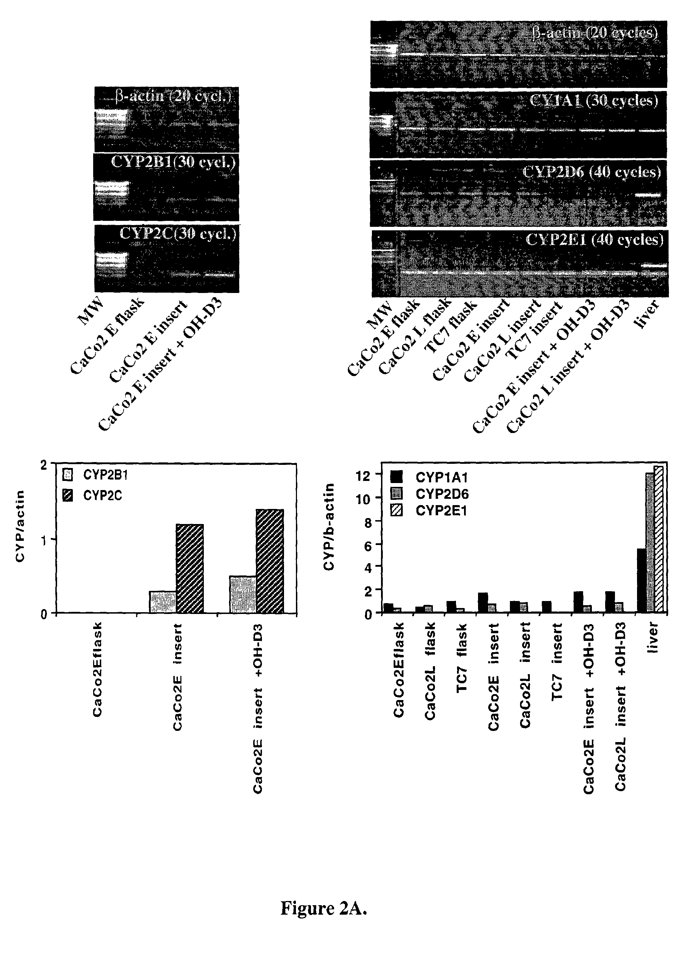

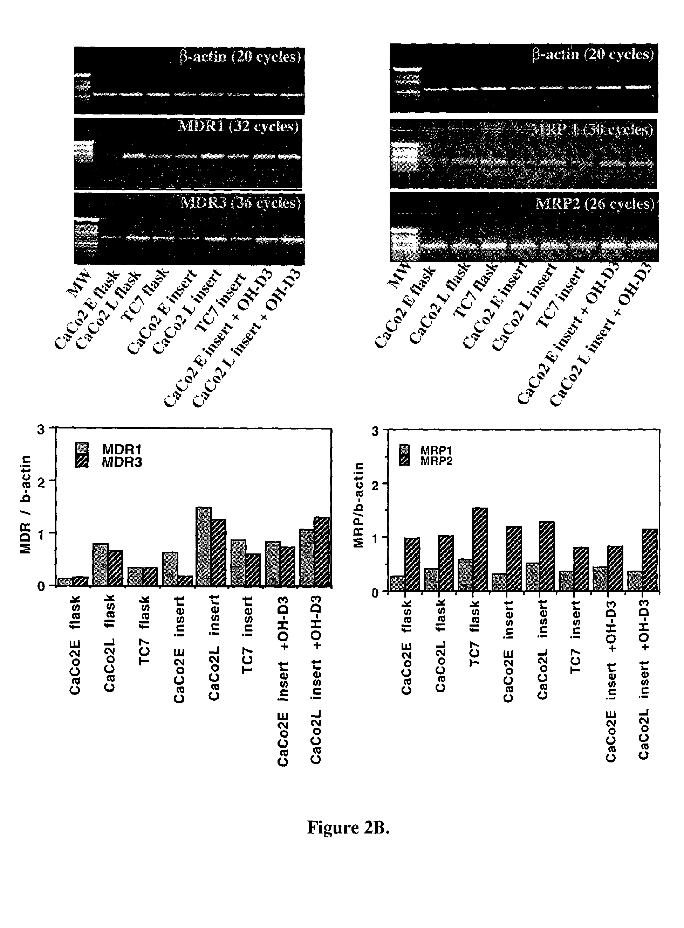

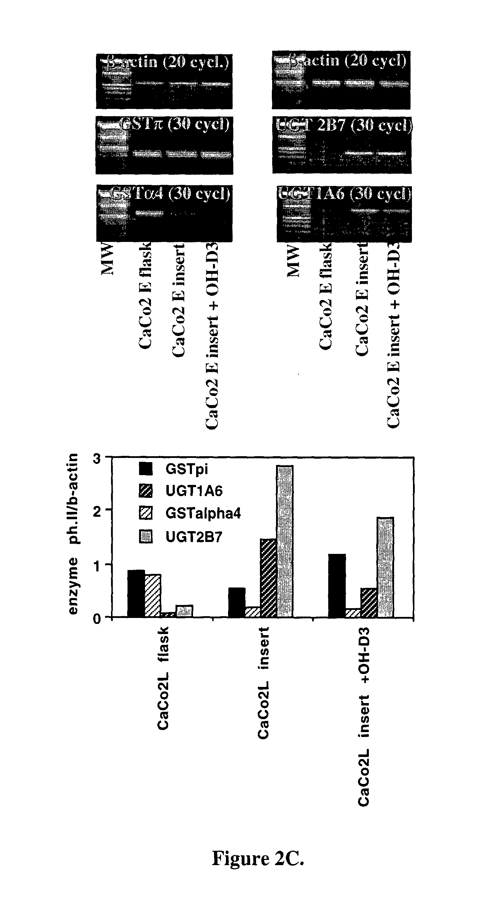

***Figure 2:*** Expression of mRNA for:
   A) phase I metabolisation enzymes (CYP1A1, 2B1, 2C, 2D6, 2E1), B) phase II metabolistation enzymes (GSTπ, α, UGT 1A6, 2B7), and C) phase III metabolisation enzymes (MDR1, MDR3, MRP1, MRP2).
   Specific primers for each enzyme were used for a RT-PCR experiment on cDNA from CaCo2E and CaCo2L cells grown on flasks or on PET inserts in synthetic BDM medium in the presence or absence of 2.5 µM 25-hydroxyvitamin D3. Ethidium bromide stained agarose gels were analysed with NIH Image with "gel plotting" macro: intensity of each band was divided by the intensity of the corresponding band of b-actin and results were collected in the graph enclosed.
***Figure 3:*** Immunoblot analysis of CYP3A.
   CaCo2 cells were grown in flasks or on PET inserts in synthetic BDM in the presence or absence of 2.5 µM 25-hydroxyvitamin D3. Western blot analysis was driven like described in the Experimental procedures.
***Figure 4:*** Metabolisation of testosterone in 6β-hydrotestosterone in CaCo2 cells.
   Cells were grown on PET inserts in synthetic BDM in the presence or absence of 5 µM 25-hydroxyvitamin D3. Testosterone (200 µM) was added at the apical (A) compartment for the study of the passage from the apical to the basolateral pole (A-B) and at the basolateral (B) compartment for the study of the passage from the basolateral pole to the apical pole (B-A).

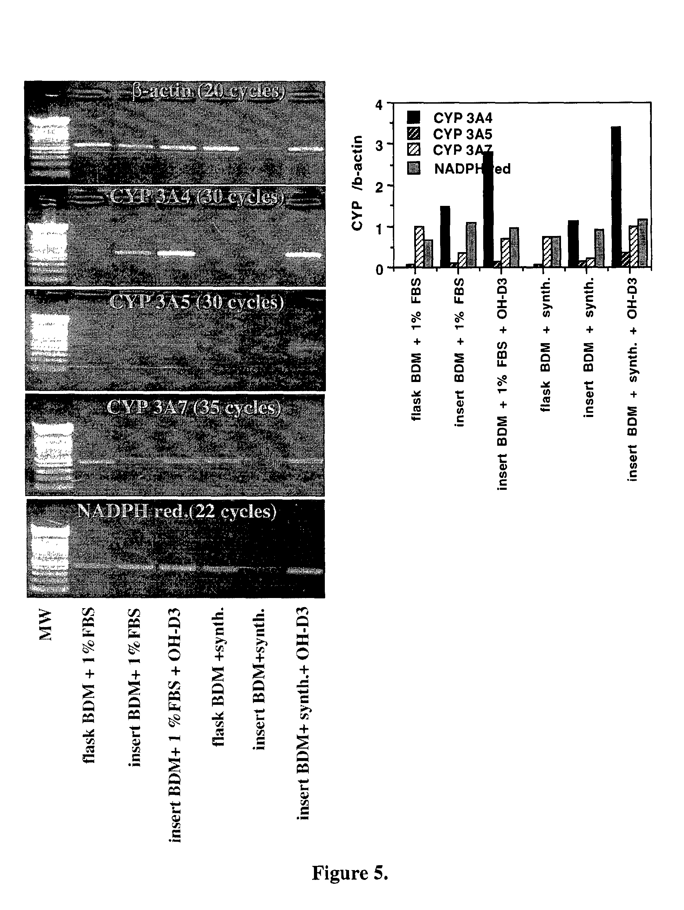

***Figure 5:*** Effect of the presence of serum during cultivation of CaCo 2 E cells on the expression of mRNA for CYP3A4, CYP3A5, CYP3A7, NADPH cytochrome P450 reductase and β-actin.
   Specific primers for CYP3A4, CYP3A5, CYP3A7 and NADPH cytochrome P450 reductase were used for a RT-PCR experiment on cDNA from CaCo 2 E grown on flasks or on PET inserts in synthetic BDM or in BDM supplemented with 1% FBS medium, in the presence or absence of 2.5 µM 25-hydroxyvitamin D3. Ethidium bromide stained agarose gels were analysed with NIH Image with "gel plotting" macro: intensity of each band was divided by the intensity of the corresponding band of β-actin and results were collected in the graph enclosed.
***Figure 6:*** Effect of different microporous substrates on the expression of the mRNA for CYP3A4 and β-actin by CaCo 2 E cells.
   Specific primers for CYP3A4 were used for a RT-PCR experiment on cDNA from CaCo 2 E grown in polystyrene flask or grown on inserts with a PTFE, PET, PC or Anopore™ inorganic membrane or on PET film in synthetic BDM medium in the presence or absence of 2.5 µM 25-hydroxyvitamin D3. Ethidium bromide stained agarose gels were analysed with NIH Image with "gel plotting" macro : intensity of each band was divided by the intensity of the corresponding band of β-actin and results were collected in the graph enclosed.

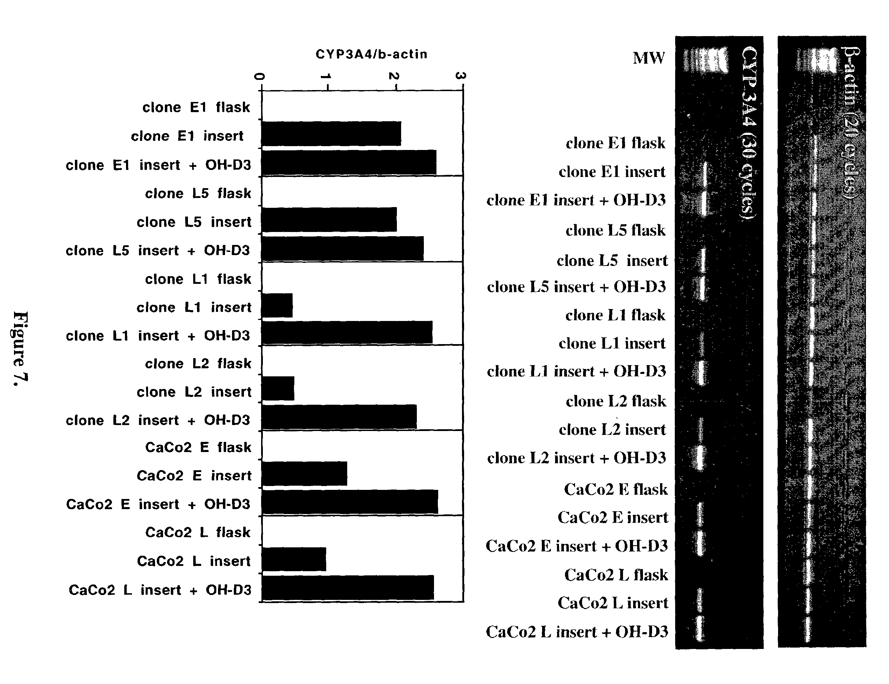

***Figure 7:*** Expression of mRNA for CYP3A4 and β-actin in clones isolated from parental CaCo 2 cells.
   Specific primers for CYP3A4 were used for a RT-PCR experiment on cDNA from CaCo2 E, CaCo2 L, clones of CaCo 2 E (clone E1) and CaCo 2 L (clone L1, clone L2 and clone L5) grown on flasks or PET inserts in synthetic BDM medium in the absence or presence of 2.5 µM of 25-hydroxyvitamin D3. Ethidium bromide stained agarose gels were analysed with NIH Image with "gel plotting" macro : intensity of each band was divided by the intensity of the corresponding band of β-actin and results were collected in the graph enclosed.

### EXAMPLES

### Example 1 : Cells and media

CaCo 2 cells "late passages" (noted as CaCo 2 L and used from passage 182 to 208) or "early passages" (noted as CaCo 2 E and used from passage 68 to 91) were obtained from American Type Culture Collection (Rockville, MD, USA). TC7 cells (used from passage 30 to passage 41), a clone of CaCo 2 cells are a gift from Dr. A. ZWEIBAUM (Laboratoire d'Oncogénétique, Hôpital Amboise Paré, Paris, France).

Iscove's modified Dubelcco's medium, HAM F-12 medium, NCTC-135 medium, L-glutamine, mixture of trace elements, MEM non essential amino acids, Random primers and Superscript II kit came from Gibco BRL (Life Technologies Ltd, Paisley, UK). Albumin complexed to linoleic acid, ethanolamine, insulin, testosterone, triiodothyronine (T3), hydrocortisone, dithiotreitol (DTT), phenylmethylsulfonyl fluoride (PMSF) and 25-hydroxycholecalciferol (hydroxyvitamin D3) were obtained from Sigma Chemical Co (St. Louis, MO, USA). Fetal bovine serum (FBS) was purchased from Bioproducts (Verviers, Belgium). Epidermal growth factor (EGF), DNA molecular weight marker XIV, deoxynucleoside triphosphate set came from Boehringer Mannheim (Mannheim, Germany). Bovine dermal type I collagen was purchased from Cellon (Strassen, Luxembourg). Primers for CYP 3A4, CYP 3A5, CYP 3A7, MDR1, MDR3, NADPH cytochrome P450 reductase and β-actin were synthesized by Eurogentec (Seraing, Belgium); the Gold Star "Red" kit and TRI Insta Pure came from the same origin. Antibody to human CYP3A (WB-MAB-3A) was purchased from Gentest (Woburn, MA, USA). PVDF membrane Hybond Pand ECL detection system come from Amersham (Little Chalfont, England). Bio Rad protein assay reagent was obtained from Bio Rad (München, Germany). Cyclic trimers of PET are a gift from Pr. R. Legras (Laboratoire de chime et physique des hauts polymeres; same university, Louvain-la-Neuve, Belgium).

### Example 2: Methods

### 1. Preparation of medium.

The BDM (basal defined medium) used for cultivation of CaCo 2 cells consists of a mixture of IMDM (Iscove's modified Dubelcco's medium), HAM F-12 medium and NCTC-135 medium in a 5:5:1 (v:v:v) ratio, supplemented by glucose up to a concentration of 16 mM, glutamine up to 6 mM, Hepes up to 11 mM, 3 g/L of sodium bicarbonate, 50 µM ethanolamine. The synthetic BDM medium consists of the BDM medium as defined above supplemented by 1 µg/ml insulin, 10 µg/ml serum albumin complexed to linoleic acid (or from 10 to 50 µg/ml of vegetable proteins hydrolysate), 100 µM hydrocortisone, 2 nM T3, 1 ng/ml EGF and a mixture of non essential amino acids and trace elements (Mn, Mo, Ni, Si, Sn, V), (Schneider, 1989; Halleux and Schneider, 1991).

The BDM 1 % FBS consists of BDM as above but supplemented with 1 % (v:v) of fetal bovine serum.

### 2. Cell cultivation.

CaCo 2 and TC7 cells are routinely grown (Halleux and Schneider, 1991; Sergent-Engelen *et al*., 1993) in tissue culture treated polystyrene flasks (175 cm², Greiner Labortechnik, Frickenhaussen, Germany). At confluence, the cells are detached by trypsin-EDTA (500 mg/L trypsin 1:250, 200 mg/L EDTA) and seeded on inserts containing at their bottom either (i) a poly(etylene terephtalate) film (11 µm thickness) or membrane (11 µm thickness, *ca*. 1.6 10⁶ pores of *ca*.or 0.45 or 1 or 3 µm/cm² Whatman SA, Louvain-la-Neuve, Belgium), (ii) a polycarbonate membrane (9 µm thickness, ca. 1.6 10⁶ pores of ca. or 0.45 or 1 or 3 µm/cm²; Whatman SA, Louvain-la-Neuve, Belgium) or (iii) an inorganic membrane Anopore™ (pore size: 0.2 µm; Nunc, Life Technologies Ltd, Paisley, UK) or (iv) polyester membrane (pore size : 0.4 or 3 µm; Corning Costar, Acton, MA, USA) or (v) polytetrafluoroethylene (pore size : 0.4 µm, Millipore Corp., Bedford,MA, USA). These substrates were coated or not with collagen Type I (33 µg/ml). Inserts were inoculated with 160,000 CaCo 2 cells/cm².

The culture in inserts are maintained for 2 weeks after confluence. The medium is replaced 3 times a week. For some experiments, the medium was supplemented with 2.5 µM 25-hydroxyvitamin D3 after confluence and for 2 weeks.

### 3. Isolation of clones.

Clones were obtained from the parental CaCo 2 cell line (E or L) by the limiting dilution technique. A dilute suspension of CaCo 2 cells was distributed into the wells of a 96-microplate such that each well was expected to receive only 1 cell. The cells from wells which appear to contain only a single colony were further propagated.

### 4. RT-PCR.

First, selective primers were chosen using Clustal W 1.7 program (Thomson *et al.,* 1994) and tested with the PCR simulation PCR Amplify 1.2. (Bill Engels© 1992. University of Wisconsin, Madison, WI 53706). The sequences of these primers are collected in Table 1.

Experimentally, at the end of the culture, cells were gently scraped, homogenized in 1 ml of TRI Insta Pure reagent and stored at -70°C. Chloroform (200 µl) was added to the cells homogenized in TRI Insta Pure (1ml) to extract total RNA. After centrifugation (15 min, 14,000 rpm, 4°C), the aqueous phase (600 µl) was isolated and isopropanol (500 µl) added to precipitate RNA. After centrifugation (15 min, 14,000 rpm, 4°C), the precipitate was rinsed with 75% ethanol (1 ml), dried at room temperature and, finally, solubilized in water.

Total RNA (2 µg) undergoes then reverse transcription in 20 µl of transcription buffer (50 mM Tris-HCI, at pH 8.3 containing 75 mM KCI, 3 mM MgCl₂, 10 mM DTT, 0.5 mM dNTP, 150 ng of random primers) with 200 U of Superscript RT reverse transcriptase at 42°C for 1h, followed by heating to 70°C for 15 min and immediate cooling on ice.

The reverse transcription mixture (1 µl) was amplified by PCR in 40 µl of buffer (75 mM Tris-HCI, at pH 9.0 containing 20 mM (NH₄)₂SO₄, 0.01% (w:v) Tween 20, 1.5 mM MgCl₂, 0.2 mM dNTP) with 0.2 µM of sense and anti-sense primers (see sequence, Table1) and 0.8 U of GoldStar DNA polymerase. The PCR was run at 94°C for 1 min, at hybridisation temperature (see Tₕ, Table 1) for 1 min and at 72°C for 1 min. These three steps were repeated (see N° cycles, Table 1). Amplification was terminated by a final incubation at 72°C for 5 min. The PCR products (20 µl) were separated on 1% agarose gels and visualized by ethidium bromide staining. PCR experiments using RNA not reverse transcribed, were run as negative controls to exclude amplification of genomic DNA. Molecular weight of PCR-products (see MW, Table 1) were checked with DNA molecular weight marker XIV. As an internal control a sample of each first strand cDNA was treated with primers amplifying for human β-actin. Results were treated with NIH Image 1.55 program (Wayne Rasband, National Institutes of Health, USA) with the gel plotting macro.

### 5. Western blot.

At the end of the culture, cells were gently scraped and resuspended in storage buffer (100 mM potassium phosphate at pH 7.4 containing 1 mM of EDTA, 20 % of glycerol, 1 mM of DTT and 2 mM of PMSF) and lysates were generated by sonication. Protein concentrations were measured according to the method of Bradford (Bradford, 1976) using bovine serum albumin as standards. For immunodetection of CYP3A, 50 µg of lysate were resuspended in Laemmli sample preparation buffer, heated for 5 min at 100°C and submitted to electrophoresis on a 10 % (w/v) polyacrylamide gel containing 0.1 % sodium docecyl sulfate. Proteins were transferred to PVDF membrane Hybond P and developed sequentially with monoclonal antibody to human CYP3A (WB-MAB-3A) and peroxidase conjugated anti-mouse IgG and then developed with the Amersham ECL + detection system.

### 6. Metabolisation of testosterone.

Inserts were washed twice with fresh medium and placed into a 6-wells plate. Respectively 2.8 ml (lower compartment, basolateral pole) and 1.8 ml (upper compartment, apical pole) of medium were added. Testosterone (200 µM) was added at the apical or at the basolateral side. After 1 h, 2.8 ml (lower) and 1.8 ml (upper) medium of each compartment were removed. Metabolisation products were extracted from the medium with dichloromethane, evaporated and solubilised in a mixture of methanol/water/acetonitrile (39/60/1), the moving phase. Samples are analysed by HPLC system with an UV detector.

### Example 3. Expression of CYP3A in CaCo2 cells.

### 1. CYP3A mRNA.

Our interest is focused on CYP3A4 but there is a large sequence homology between CYP3A4, CYP3A5 and CYP3A7. Furthermore, CYP3A4 and CYP3A5 have, largely, the same substrates and inhibitors. Nevertheless CYP3A5 doesn't seem to be inducible like CYP3A4 is. NADPH cytochrome P450 reductase regenerates CYP3A4 in the oxidised state after metabolisation reaction and the presence of the mRNA coding for this enzyme was also tested. Finally, in the enterocytes, extrusion systems like P-glycoprotein (MDR1, MDR3) or multidrug resistance associated proteins (MRP1, MRP2, MRP3, ...) seem implicated in synergy with phase I (CYP 1A1, 3A4, 3a5, 3A7, 2D6, 2E1, 2B1, 2C, ...) and phase II (GSTα4, GSTπ, UGT 1A6, 2B7) activities in the control of bioavailability of a lot of xenobiotics. Then it was justified to test, in our CaCo2 cells, the presence of the gene coding for all these proteins.

### 2. Subtypes of CaCo 2 cells.

In a first set of experiments, cDNA's were synthesized from RNA of CaCo 2 cells at early and late passages as well as from TC7 cells grown either for 3 days up to confluence on flasks or for 3 weeks up to full differentiation on inserts containing a microporous membrane. The cultivation was performed in a synthetic medium in the presence or not of 2.5 µM of 25-hydroxyvitamin D3 during the 2 weeks after confluence. PCR were made following the experimental procedure described above (Table 1).

The results, shown in figure 1, indicate that CaCo2 cells grown on flasks don't express the CYP3A4 mRNA. When CaCo 2 cells (late or early passage) are grown in inserts on a PET-microporous membrane, the CYP3A4 mRNA is detected and, furthermore, may still be induced by the addition of 25-hydroxyvitamin D3 to the nutritive medium during cell differentiation. The CYP3A4 mRNA is not expressed in TC7 cells whatever the culture conditions.

The CYP3A5 mRNA is detected in CaCo 2 cells at early and late passages and in TC7 cells but without increase after cultivation of the cells on inserts. In addition, the CYP3A5 mRNA is not induced by the presence of 25-hydroxyvitamin D3 in the nutritive medium. The CYP3A7 mRNA is poorly expressed in all conditions and cells tested and couldn't be induced by the addition of 25-hydroxyvitamin D3 to the nutritive medium. Finally, NADPH cytochrome P450 reductase mRNA is strongly expressed in all conditions and cells tested but doesn't appear to be induced by 25-hydroxyvitamin D3.

The results indicate that CYP3A4 mRNA may be expressed in CaCo 2 cells cultivated in serum free medium and that the expression is enhanced by the presence of 25-hydroxyvitamin D3 during the differentiation phase. CYP3A4 mRNA is the only one to be inducible in the conditions used during this experimentation. CYP3A5 (present in colonic cells), CYP3A7 (fetal isoform) and NADPH cytochrome P450 reductase (regenerating enzyme of cytochrome P450) seem to be unaffected by the addition of 25-hydroxyvitamin D3.

### 3. Other genes of proteins implicated in metabolisation of xenobiotics.

The presence of mRNA coding for other phase I metabolisation enzymes was tsted (figure 2A). After that, oter PR-PCR were made to check the presence of mRNA coding for phase II (figure 2B) and phase III (figure 2C) metabolisation enzymes. RT-PCR reveals that expression of CYP2C and 2B1, UGT 1A6 and 2B7 and MDR1 is higher when cells are grown on PET than on solid substrates whereas that of CYP1A1 and 2D6, GSTπ, MDR3, MRP1, and MRP2, is not significantly affected. The addition of hydroxyvitamin D3 during cell differentiation doesn't increase the expression of the RNA's coding for these proteins. Other tests have confirmed the presence of mRNA coding for LRP and MRP3 in CaCo2 cells grown on PET membrane (data not shown) and the absence of mRNA coding for CYP2E1.

### 4. Detection of CYP3A protein by Western blot.

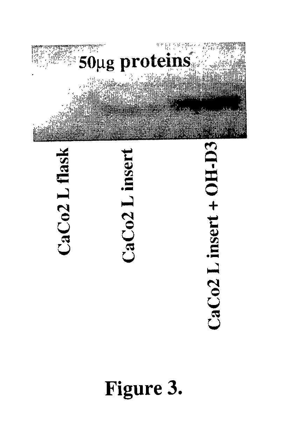

Concurrently to RT-PCR, we have tried to detect the expression of the protein of CYP3A4 by Western blot using antibodies to CYP3A. Nevertheless, the lack of real specific antibody to CYP3A4 complicates this task. Preliminary results allow to detect more protein reacting with anti CYP3A antibodies in cells cultivated with 25-hydoxyvitamin D3 during the differentiation phase (figure 3). No protein was recognized by the antibody when cells are grown on flasks. Since PCR results indicate that 25-hydoxyvitamin D3 does not induce the CYP3A5 and CYP3A7 mRNAs, these results strongly suggest that the increase of CYP3A protein could result from an increase in the expression of CYP3A4 protein.

### 5. Metabolisation of testosterone.

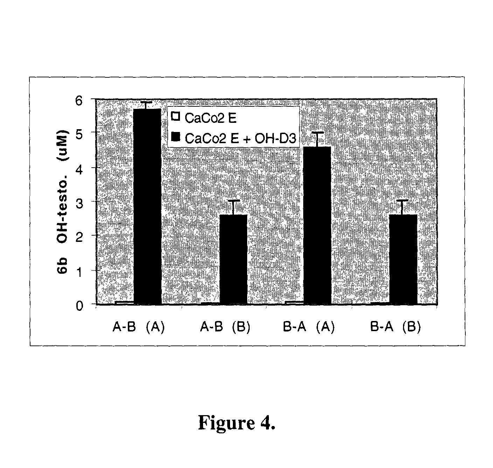

Other results concerning the metabolisation of testosterone (substrate of CYP3A) in 6β-hydroxytestosterone show an enhanced CYP3A activity when cells are grown in the presence of 25-hydroxyvitamin D3 (figure 4). Like for detection of protein by an immunological way, there is a problem of substrate specificity. All the substrates of CYP3A4 are metabolized by CYP 3A5 and often by CYP3A7. But the increase of CYP3A activity could be due to CYP3A4 activity because, at the RNA level, 25-hydroxyvitamin D3 isn't an inducer of CYP3A5 and CYP3A7 expression.

### Example 4. Culture conditions.

### 1. Effect of serum.

The use of serum-free, hormono-defined, synthetic medium generally improves the reproducibility of cell cultures. In the serum, the concentration of a lot of components varies and could affect both the proliferation and the differentiation of CaCo 2 cells. We have tested, in our culture system, the effect of the addition of fetal bovine serum to the nutritive medium. CaCo 2 E cells were grown on flasks or on PET inserts in the presence or absence of 25-hydroxyvitamin D3. As illustrated in figure 5, RT-PCR indicates that the addition of FBS to BDM does not affect significantly the amount of CYP3A4 mRNA. As above, the expression of CYP3A4 appears higher after the addition of 25-hydroxyvitamin D3 to the nutritive medium.

### 2. Microporous membranes.

Another set of experiments was addressed to determine whether the chemical nature of the microporous substrate could influence the expression of CYP3A mRNA and, more particulary, CYP3A4. Other microporous supports were compared to track etched PET membranes (pore size : 1 µm) previously used (figures 1,2,5) : (i) PET membranes (pore size 0.45 and 3 µm) from the same origin (Whatman), (ii) polyester membrane, in fact PET membrane from an other origin (Costar), called hereafter PET*, (iii) polycarbonate (PC) membrane also produced by the track etching process (pore sizes : 0.45, 1 or 3 µm), (iv) Anopore™ "inorganic" membranes (pore size : 0.2 µm) and, finally, polytetrafluoroethylene (PTFE) with pores size of 0.4 µm.

CaCo 2 cells were grown on these membranes in the same culture conditions in the absence of serum. In the same set of experiments, we have also studied the influence of a PET membrane put at the bottom of the well (under the insert) and separated from the cells by a PC membrane on which they were seeded as well as the effect of the addition of insoluble cyclic oligomers (largely trimers) of PET (200 µg/ml) in the nutritive medium. Collected RNA were amplified by PCR, as above, to detect the presence of mRNA for CYP3A4 and β-actin (figure 6).

Surprisingly, only the PET track etched membrane and the "inorganic" Anopore™ membrane allow the expression of CYP3A4 mRNA although they completely differ by chemical nature, physicochemical process of preparation and pore size. Furthermore, the PC membrane whose chemical matrix is closer to PET, is made by the same track etching process and have similar porosity (pore size and pore number) does not allow significant level of expression of the CYP3A4 mRNA.

This observation could be explained if we consider that the expression of CYP3A4 mRNA is modulated by two factors : (i) the pore size and (ii) the chemical nature of the substrate.

Figure 6A shows indeed that the level of CYP3A4 mRNA increases as the pore diameter of the PET membrane decrease from 3 to 0.45 µm. It is important to note that two PET from different origin (PET and PET*) induce a similar behaviour even if cells grown on PET* seems to express more CYP3A4 mRNA without addition of hydroxyvitamin D3 in the culture medium (figure 6B). In addition, CaCo 2 cells cultivated on Anopore membrane (0.2 µm) also have a high level of expression of CYP3A4 (figure 6D).

Note that the chemical nature of the PET also influences the expression of CYP3A4 in CaCo 2 cells. Cells grown on PET membrane with pore size of 0.45 µm express more CYP3A4 mRNA than cells grown on membrane characterized by the same pore size but made of PTFE (on which no expression is detected) or PC (less expression), (figure 6B). More, cells grown on a PC membrane in the presence of a PET membrane at the bottom of the well express more CYP3A4 mRNA than in its absence (figure 6B). Finally, the addition of insoluble oligomers of PET, to the nutritive medium seems to have a comparable inductive effect (figure 6B) as a direct cultivation of CaCo 2 cells on a PET membrane.

When CaCo2 cells are cultivated on polystyrene flask (3 days after confluence) no expression of CYP3A4 is found but when they are grown on a PET film (2 weeks after confluence), CYP3A4 mRNA is present (figure 6C).

Finally, it should be emphasized that the addition of 25-hydroxyvitamin D3 allows to increase significantly the expression of CYP3A4 mRNA in cells grown on all the substrates tested (figure 6D).

### Example 5. Isolation of clones of CaCo 2 cells with high expression levels of CYP3A4.

In order to obtain more homogeneous subpopulations of CaCo 2 cells, clones were isolated by the limiting dilution technique. Clones were then grown, as above, in flasks or on inserts, in the presence or absence of 25-hydoxyvitamin D3 in the nutritive medium. After isolation of RNA and synthesis of cDNA, PCR was made with selective primers for CYP3A4.

Figure 7 indicates that clones L5 and E1 contain obviously more CYP3A4 in the absence of induction with 25-hydroxyvitamin D3 than clones L1, L2 and parent CaCo 2 cells (E or L). When cells are induced with hydroxyvitamin D3, an additional increase in expression of CYP3A4 is observed for clones L5 and E1, reaching the same level as with the other cell populations.

The cell line corresponding to clone L5, further specified as CaCo 2-BT-01, has been deposited at the Belgian Coordinated Collection of Microorganisms (LMBP) on April 10, 2000.

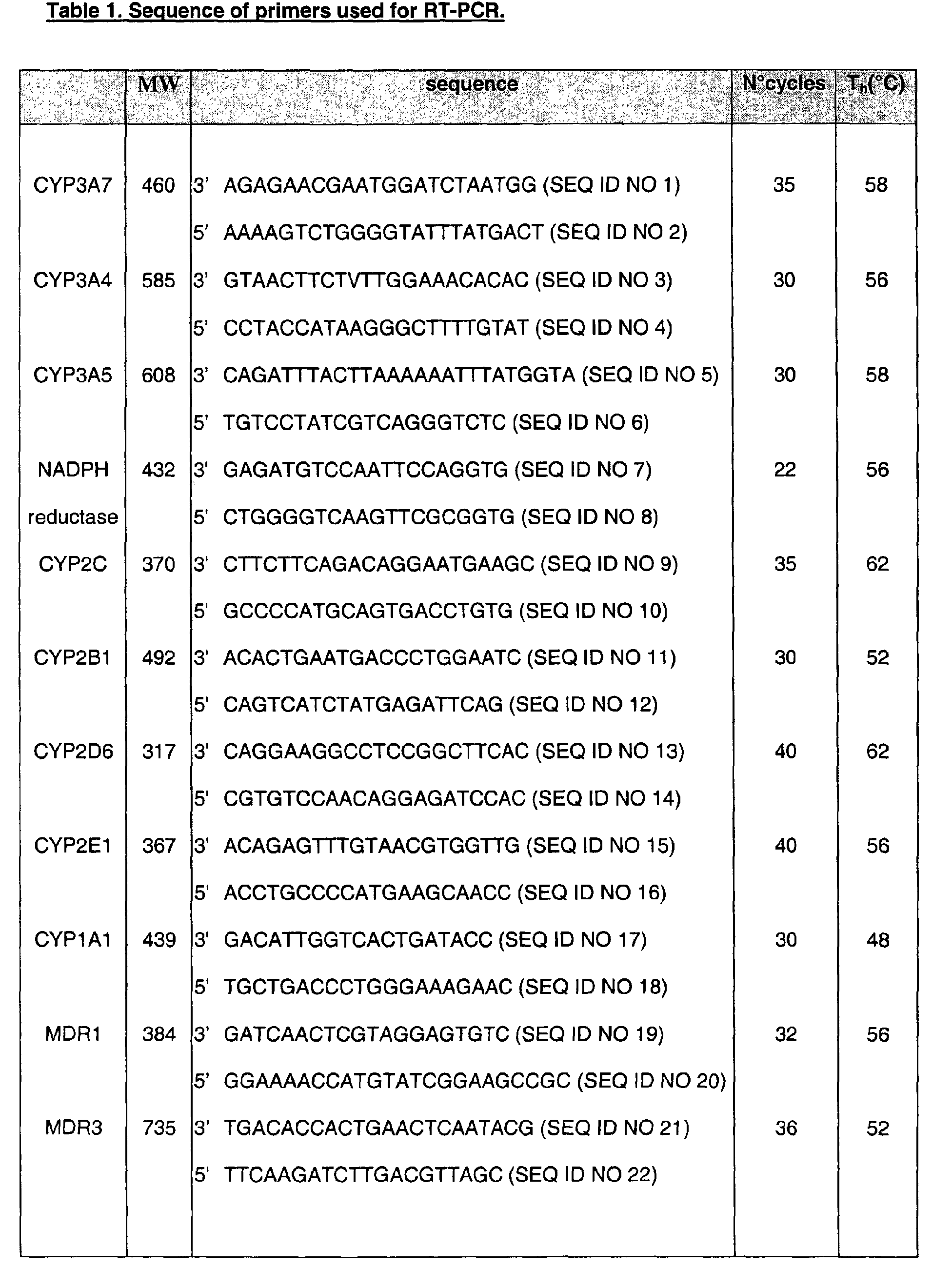

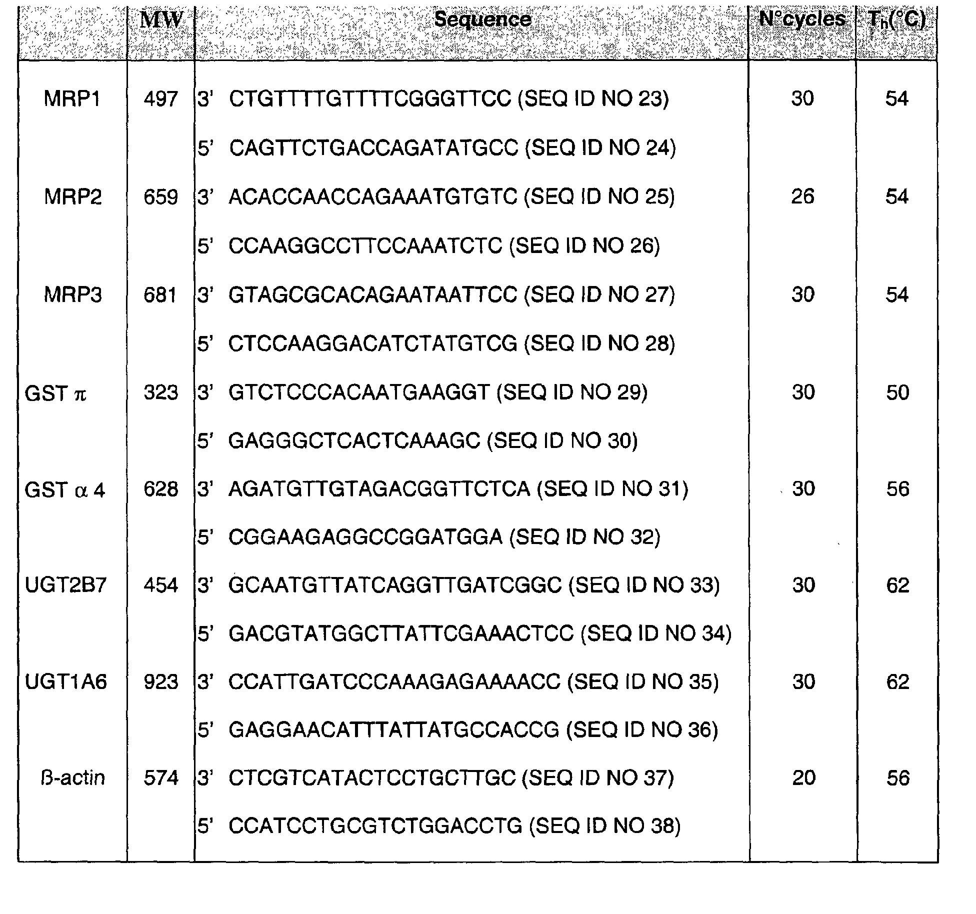

### REFERENCES

Artursson, P., K. Palm and K. Luthman. CaCo 2 monolayers in experimental and theoretical predictions of drug transport. *Adv*. *Drug Deliv*. *Rev.* **22:**67-84 (1996).

Bailey, C.A., P. Bryla and A. Waseem Malick. The use of the intestinal epithelial cell culture model, CaCo 2, in pharmaceutical development. *Adv. Drug Deliv*. *Rev.* **22**:85-103 (1996).

Bradford, M.M. . A rapid and sensitive method for the quantification of microgram quantities of protein utilizing the principle of protein-dye binding. *Anal. Biochem.* **72**:248-254 (1976).

Carrière, V., T. Lesuffleur, A. Barbat, M. Rousset, E. Dussaulx, P. Costet, I. de Waziers, P. Beaune and A. Zweibaum. Expression of cytochrome P450 3A in HT29-MTX cells and CaCo 2 clone TC7. FEBS Lett. 355:247-250 (1994).

Crespi C.L., B.W. Penman and M. HU. Development of CaCo 2 cells expressing hih levels of cDNA derived cytochrome P4503A4. *Pharm*. *Res.* **13:**1635-1641 (1996).

Gan L.-S.L., M.A. Moseley, B. Khosla, P.F. Augustijns, T.P. Braddshaw, R.W. Henderen and D.R. Thakker. CYP3A-like cytochrome P450-mediated metabolism and polarized efflux of cyclosporin A in CaCo 2 cells. *Drug Metab. Dipos*. **24**:344-349(1996).

Halleux C. and Y.J. Schneider. Iron absorption by intestinal epithelial cells: CaCo 2 cells cultivated in serum-free medium, on polyethylene terephtalate microporous membranes, as an *in vitro* model. *In vitro Cell Dev*. *Biol.* **27A**:293-302 (1991).

Hillgren, K.M., A. Kato and R.T. Borchadt. *In vitro* systems for studying intestinal drug absorption. *Med*. *Res*. *Rev*. **15**:83-109 (1995).

Hu M., Y. Li, Ch.M. Davitt, S.-M. Huang, K. Thummel, B.W. Penman and C.L. Crespi, *Pharmac*. *Res.,* **16,** 1352-1359 (1999).

Kolars, J.C., W.M. awni, R.M. Merion and P.B. Watkins. Identification of rifampin-inducible P450 IIIA4 (CYP3A4) in human small bowel enterocytes. *J*. *Clin. Invest*. **90**:1871-1878 (1992).

Lown, K.S., D.G. Bailey, R.J. Fontana, S.K. Janardan, C.H. Adair, L.A. Fortlage, M.B. Brown, W. Guo and P.B. Watkins. Grapefruit juice increases felodipine oral availibility in humans, by decreasing intestinal CYP3A protein expression. *J*. *Clin*. *Invest.* **99**:2545-2553 (1997).

Shimada, T. and F.P. Guengerich. Evidence for cytochrome P450NF, the nifedipine oxydase, being the principal enzyme involved in the bioactivation of aflatoxins in human liver. *Proc*. *Natl. Acad. Sci. USA* **86**:462-465 (1989).

Schmiedlin-Ren P., K.E. Thummel, J.M. Fisher, M.F. Paine, K.S. Lown and P.B. Watkins. Expression of enzymatically active CYP3A4 by CaCo 2 cells grown on extracellular matrix-coated permeable supports in the presence of 1□,25-Dihydroxyvitamin D3. *Mol*. *Pharmacol.* **51**:741-754 (1997).

Schneider Y-J., Optimisation of hybridoma cell growth and monoclonal antibody secretion in a chemically defined, serum- and protein-free culture medium. *Immunol*. *Methods* 116: 65-77 (1989).

Sergent T., V. Delistrie and Y.J. Schneider. Phase I and II biotransformations in living CaCo 2 cells cultivated under serum-free conditions. *Biochem*. *Pharmac.* **46** (8):1393-1401 (1993).

Skarosi S., C. Abraham, M. Bisonnette, B. Scagglione-Sewell, M.D. Sitri, T.A. Brasitus. *Gastroenerology*, **112** (4): A658 (1997).

Thomson, J.D., D.G. Higgins and T.J. Gibbson. *Nucleic Acid Res.* **22**:4673-4680(1994).

Watkins, P.B., S.A. Wrighton, EG. Schuetz, D.T. Molowa and P.S. Guzelian. Identification of glucocorticoide-inducible cytochrome P450 in the intestinal mucosa of rats and man. *J. Clin*. *Invest.* **80:**1029-1036 (1987).

## Claims

1. A CaCo 2 cell line showing an enhanced expression of CYP3A4 without the addition of an inducer for CYP3A4 expression.

2. A CaCo 2 cell line according to claim 1 showing an enhanced expression of CYP3A4 **characterized in that** it reaches at least the same level of CYP3A4 expression of the cell line deposited under the name CaCo 2-BT-01 at the Belgian Coordinated Collection of Microorganisms (LMBP) on April 10, 2000.

3. A CaCo 2 cell line according to claim 1 as deposited under the name CaCo 2-BT-01 at the Belgian Coordinated Collection of Microorganisms (LMBP) on April 10, 2000.

4. A method of growing CaCo2 cells according to any of claim 1 to 3 comprising growing said cells without the addition of an inducer for CYP3A4 expression.

5. A method of growing CaCo2 cells comprising the steps of:
- providing at least:
a) a CaCo 2 cell line according to any of claims 1 to 3, and,
b) a growth medium for cultivation of said CaCo 2 cells comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and,
- seeding said cells onto a permeable culture insert.

6. The method of claim 5 wherein said permeable culture insert is chosen from PET microporous membranes having a pore size of 0.4 to 3 µm and Anopore™ membranes having a pore size of 0.02 to 0.2 µm.

7. A method of growing CaCo 2 cells to allow for the enhanced expression of CYP3A4 without any addition of an inducer for CYP3A4 expression comprising growing said cells on a permeable culture insert chosen from PET microporous membranes having a pore size of 0.4 to 3 µm and an Anopore™ membranes having a pore size of 0.02 to 0.2 µm.

8. A method of growing CaCo 2 cells according to claim 7 comprising the steps of:
- providing a growth medium for cultivation of said CaCo 2 cells comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and,
- seeding said cells on a permeable culture insert.

9. An *in vitro* model for screening the bioavailability of pharmaceutical compounds, xenobiotics or environmental pollutants, comprising a CaCo 2 cell line according to any of claims 1 to 3, seeded onto a permeable culture insert.

10. An *in vitro* model for screening the bioavailability of pharmaceutical compounds, xenobiotics or environmental pollutants comprising:
a) a CaCo 2 cell line according to any of claims 1 to 3,
b) possibly a mucus secreting cell line, and,
c) a growth medium comprising a mixture of IMDM-medium, HAM F-12 medium and NCTC-135 medium in a 5:5:1 (v:v:v) ratio, supplemented by glucose up to a concentration of 16 mM, glutamine up to 6 mM, Hepes up to 11 mM, sodium bicarbonate up to 3 g/L, ethanolamine up to 50 µM, insulin up to 1 µg/ml, serum albumin complexed to linoleic acid up to 10 µg/ml, hydrocortisone up to 100 µM, triiodothyronine up to 2 nM, epidermal growth factor up to 1 ng/ml and a mixture of non essential amino acids, and,
d) a permeable culture insert.

11. The *in vitro* model according to claim 9 or 10 wherein the permeable culture insert is chosen from PET microporous membranes having a pore size of 0.4 to 3 µm and Anopore™ membranes having a pore size of 0.02 to 0.2 µm.

12. An *in vitro* model for screening the bioavailability of pharmaceutical compounds, xenobiotics or environmental pollutants comprising:
a) a CaCo 2 cell line,
b) possibly providing a mucus secreting cell line, and,
c) providing a method of growing said CaCo 2 cells to allow for the enhanced expression of CYP3A4 according to claim 7 or 8.

13. The *in vitro* model according to any of claims 9 to 12 further comprising the use of an inducer for CYP3A4 expression.

14. A method of growing a CaCo 2 cell line according to any of claims 1 to 3 comprising treating said cells with an inducer for CYP3A4 expression.

15. The method according to claim 14 comprising the steps of:
- providing at least:
a) a CaCo 2 cell line according to any of claims 1 to 3,
b) possibly a mucus secreting cell line, and,
c) an inducer of CYP3A4 expression, and,
- seeding said cells onto a permeable culture insert, and,
- treating said cells with said inducer.

16. The method according to claim 14 or 15 wherein said permeable culture insert is chosen from PET microporous membranes having a pore size of 0.4 to 3 µm and Anopore™ membranes having a pore size of 0.02 to 0.2 µm.

17. The method according to any of claims 13 to 16 wherein said inducer is chosen from a vitamin D3 derivative or sodium butyrate.


### Annex to the application documents - subsequently filed sequences listing

SEQUENCE LISTING

<110> UNIVERSITE CATHOLIQUE DE LOUVAIN

<120> CULTURE CONDITIONS ALLOWING TO MODULATE THE EXPRESSION
      OF CYP3A4 IN CaCo2 CELLS

<130> UCL-004-EP

<140> 00870108.8
<141> 2000-05-17

<160> 38

<170> PatentIn Ver. 2.1

<210> 1
<211> 22
<212> DNA
<213> Artificial Sequence

<220>
<223> Description of Artificial Sequence:oligonucleotide

<400> 1
agagaacgaa tggatctaat gg                                          22

<210> 2
<211> 23
<212> DNA
<213> Artificial Sequence

<220>
<223> Description of Artificial Sequence:oligonucleotide

<400> 2
aaaagtctgg ggtatttatg act                                         23

<210> 3
<211> 22
<212> DNA
<213> Artificial Sequence

<220>
<223> Description of Artificial Sequence:oligonucleotide

<400> 3
gtaacttctv ttggaaacac ac                                                22

<210> 4
<211> 22
<212> DNA
<213> Artificial Sequence

<220>
<223> Description of Artificial Sequence:oligonucleotide

<400> 4
cctaccataa gggcttttgt at                                                22

<210> 5
<211> 26
<212> DNA
<213> Artificial Sequence

<220>
<223> Description of Artificial Sequence:oligonucleotide

<400> 5
cagatttact taaaaaattt atggta                                            26

<210> 6
<211> 20
<212> DNA
<213> Artificial Sequence

<220>
<223> Description of Artificial Sequence:oligonucleotide

<400> 6
tgtcctatcg tcagggtctc                                                   20

<210> 7
<211> 20
<212> DNA
<213> Artificial Sequence

<220>
<223> Description of Artificial Sequence:oligonucleotide

<400> 7
gagatgtcca attccaggtg                                                    20

<210> 8
<211> 20
<212> DNA
<213> Artificial Sequence

<220>
<223> Description of Artificial Sequence:oligonucleotide

<400> 8
ctggggtcaa gttcgcggtg                                                    20

<210> 9
<211> 22
<212> DNA
<213> Artificial Sequence

<400> 9
cttcttcaga caggaatgaa gc                                                 22

<210> 10
<211> 20
<212> DNA
<213> Artificial Sequence

<220>
<223> Description of Artificial Sequence:oligonucleotide

<400> 10
gccccatgca gtgacctgtg                                                    20

<210> 11
<211> 21
<212> DNA
<213> Artificial Sequence

<220>
<223> Description of Artificial Sequence:oligonucleotide

<400> 11
acactgaatg accctggaat c                                                  21

<210> 12
<211> 20
<212> DNA
<213> Artificial Sequence

<220>
<223> Description of Artificial Sequence:oligonucleotide

<400> 12
cagtcatcta tgagattcag                                                    20


<210> 13
<211> 21
<212> DNA
<213> Artificial Sequence

<220>
<223> Description of Artificial Sequence:oligonucleotide

<400> 13
caggaaggcc tccggcttca c                                                  21


<210> 14
<211> 21
<212> DNA
<213> Artificial Sequence

<220>
<223> Description of Artificial Sequence:oligonucleotide

<400> 14
cgtgtccaac aggagatcca c                                                  21


<210> 15
<211> 21
<212> DNA
<213> Artificial Sequence

<220>
<223> Description of Artificial Sequence:oligonucleotide

<400> 15
acagagtttg taacgtggtt g                                                  21

<210> 16
<211> 20
<212> DNA
<213> Artificial Sequence

<220>
<223> Description of Artificial Sequence:oligonucleotide

<400> 16
acctgcccca tgaagcaacc                                                    20


<210> 17
<211> 19
<212> DNA
<213> Artificial Sequence

<220>
<223> Description of Artificial Sequence:oligonucleotide

<400> 17
gacattggtc actgatacc                                                     19


<210> 18
<211> 20
<212> DNA
<213> Artificial Sequence

<220>
<223> Description of Artificial Sequence:oligonucleotide

<400> 18
tgctgaccct gggaaagaac                                                    20


<210> 19
<211> 20
<212> DNA
<213> Artificial Sequence

<220>
<223> Description of Artificial Sequence:oligonucleotide

<400> 19
gatcaactcg taggagtgtc                                                    20

<210> 20
<211> 24
<212> DNA
<213> Artificial Sequence

<220>
<223> Description of Artificial Sequence:oligonucleotide

<400> 20
ggaaaaccat gtatcggaag ccgc                                                24

<210> 21
<211> 22
<212> DNA
<213> Artificial Sequence

<220>
<223> Description of Artificial Sequence:oligonucleotide

<400> 21
tgacaccact gaactcaata cg                                                  22

<210> 22
<211> 20
<212> DNA
<213> Artificial Sequence

<220>
<223> Description of Artificial Sequence:oligonucleotide

<400> 22
ttcaagatct tgacgttagc                                                     20

<210> 23
<211> 20
<212> DNA
<213> Artificial Sequence

<220>
<223> Description of Artificial Sequence:oligonucleotide

<400> 23
ctgttttgtt ttcgggttcc                                                     20

<210> 24
<211> 20
<212> DNA
<213> Artificial Sequence

<220>
<223> Description of Artificial Sequence:oligonucleotide

<400> 24
cagttctgac cagatatgcc                                                    20


<210> 25
<211> 20
<212> DNA
<213> Artificial Sequence

<220>
<223> Description of Artificial Sequence:oligonucleotide

<400> 25
acaccaacca gaaatgtgtc                                                    20


<210> 26
<211> 19
<212> DNA
<213> Artificial Sequence

<220>
<223> Description of Artificial Sequence:oligonucleotide

<400> 26
ccaaggcctt ccaaatctc                                                     19


<210> 27
<211> 20
<212> DNA
<213> Artificial Sequence

<220>
<223> Description of Artificial Sequence:oligonucleotide

<400> 27
gtagcgcaca gaataattcc                                                    20

<210> 28
<211> 20
<212> DNA
<213> Artificial Sequence

<220>
<223> Description of Artificial Sequence:oligonucleotide

<400> 28
ctccaaggac atctatgtcg                                                    20


<210> 29
<211> 18
<212> DNA
<213> Artificial Sequence

<220>
<223> Description of Artificial Sequence:oligonucleotide

<400> 29
gtctcccaca atgaaggt                                                      18


<210> 30
<211> 17
<212> DNA
<213> Artificial Sequence

<220>
<223> Description of Artificial Sequence:oligonucleotide

<400> 30
gagggctcac tcaaagc                                                       17


<210> 31
<211> 21
<212> DNA
<213> Artificial Sequence

<220>
<223> Description of Artificial Sequence:oligonucleotide

<400> 31
agatgttgta gacggttctc a                                                  21

<210> 32
<211> 18
<212> DNA
<213> Artificial Sequence

<220>
<223> Description of Artificial Sequence:oligonucleotide

<400> 32
cggaagaggc cggatgga                                                    18


<210> 33
<211> 23
<212> DNA
<213> Artificial Sequence

<220>
<223> Description of Artificial Sequence:oligonucleotide

<400> 33
gcaatgttat caggttgatc ggc                                              23


<210> 34
<211> 24
<212> DNA
<213> Artificial Sequence

<220>
<223> Description of Artificial Sequence:oligonucleotide

<400> 34
gacgtatggc ttattcgaaa ctcc                                             24


<210> 35
<211> 23
<212> DNA
<213> Artificial Sequence

<220>
<223> Description of Artificial Sequence:oligonucleotide

<400> 35
ccattgatcc caaagagaaa acc                                              23

<210> 36
<211> 23
<212> DNA
<213> Artificial Sequence

<220>
<223> Description of Artificial Sequence:oligonucleotide

<400> 36
gaggaacatt tattatgcca ccg                                                23

<210> 37
<211> 20
<212> DNA
<213> Artificial Sequence

<220>
<223> Description of Artificial Sequence:oligonucleotide

<400> 37
ctcgtcatac tcctgcttgc                                                    20

<210> 38
<211> 20
<212> DNA
<213> Artificial Sequence

<220>
<223> Description of Artificial Sequence:oligonucleotide

<400> 38
ccatcctgcg tctggacctg                                                    20